(19) **Europäisches Patentamt / European Patent Office / Office européen des brevets**

(11) **EP 3 170 814 B1**

(12) # EUROPEAN PATENT SPECIFICATION

(45) Date of publication and mention of the grant of the patent:
**16.09.2020  Bulletin 2020/38**

(51) Int Cl.:
*C07D 317/40* *(2006.01)*    *H01G 11/64* *(2013.01)*
*H01M 10/0567* *(2010.01)*

(21) Application number: **15822022.8**

(22) Date of filing: **23.06.2015**

(86) International application number:
**PCT/JP2015/068019**

(87) International publication number:
**WO 2016/009793 (21.01.2016 Gazette 2016/03)**

(54) **HIGH-PURITY VINYLENE CARBONATE, NONAQUEOUS ELECTROLYTIC SOLUTION, AND ELECTRICITY STORAGE DEVICE INCLUDING SAME**

HOCHREINES VINYLENCARBONAT, WASSERFREIE ELEKTROLYTLÖSUNG UND ELEKTRIZITÄTSSPEICHERUNGSVORRICHTUNG DAMIT

CARBONATE DE VINYLÈNE DE HAUTE PURETÉ, SOLUTION ÉLECTROLYTIQUE NON AQUEUSE ET DISPOSITIF D'ACCUMULATION D'ÉNERGIE ÉLECTRIQUE LA COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2014  JP 2014144374**
**10.09.2014  JP 2014184642**

(43) Date of publication of application:
**24.05.2017  Bulletin 2017/21**

(73) Proprietor: UBE Industries, Ltd.
**Ube-shi, Yamaguchi 755-8633 (JP)**

(72) Inventors:
• **ABE, Koji**
**Ube-shi**
**Yamaguchi 755-8633 (JP)**
• **ITO, Akikazu**
**Ube-shi**
**Yamaguchi 755-8633 (JP)**

• **SHIKITA, Shoji**
**Ube-shi**
**Yamaguchi 755-8633 (JP)**

(74) Representative: **Gille Hrabal**
**Brucknerstrasse 20**
**40593 Düsseldorf (DE)**

(56) References cited:
CN-A- 1 789 259       DE-A1-102005 021 965
JP-A- H11 123 302     JP-A- 2002 346 303
JP-A- 2007 284 427    JP-A- 2008 540 467
JP-A- 2010 282 760    JP-A- 2014 511 149
US-A- 3 457 279

• SCHNAUTZ, N. G. ET AL.: 'Radiation polymerization of vinylene carbonate, Report, PER-11' 1978, pages 11 - 6, XP008185483 Retrieved from the Internet: <URL:URL: https://inis.iaea.org/search/search.aspx?or ig q=RN:10432614> [retrieved on 2015-09-07]

**Description**

TECHNICAL FIELD

The present invention relates to a high-purity vinylene carbonate that does not contain impurities, a nonaqueous electrolytic solution, and an energy storage device using the same.

BACKGROUND ART

It is widely known that vinylene carbonate (hereinafter also referred to as "VC") is useful as an additive for an electrolytic solution for lithium secondary batteries, and it is known that a high-purity VC having a low content of chlorine impurities is useful as an additive of electrolytic solutions (see PTLs 1, 4, and 5). As a purification method of VC, there are proposed various methods, such as distillation, crystallization, or a combined method of the both, etc. (see PTL 2). In addition, there is known a method in which a crude vinylene carbonate is treated with urea at 140°C and then distilled, followed by purification in a static melt crystallizer (see PTL 3).

PTL 6 describes a process for the storage and transport of VC, characterized in that the VC has a stabilizer content of less than 100 ppm and a purity of 99.9 to 99.99999% and is in the solid state.

PTL 7 describes vinylene carbonate having been prepared by the chlorination of ethylene carbonate, followed by dehydrochlorination and thus vinylene carbonate having been prepared by the direct catalytic dehydrogenation of ethylene carbonate.

PTL 8 describes a method for synthesing vinylene carbonate, employing fixed bed reactor, adding catalyst, employing nitrogen, argon gas or hydrogen as diluting gas and protecting gas, the reaction temperature is 200-600 Deg C, the velocity of feeding is 1-100 g/h mlcat, the reaction pressure is 0.1-10 Mpa, getting the vinylene carbonate through vinyl carbonate catalytic dehydrogenation. The invention is characterized by the higher selectivity of reaction product, less side product, simple operation, no corrosion and pollution in reacting course.

A conventional VC containing 100 ppm or more of chlorine impurities was colored brown to yellow. By controlling it such that the content of the chlorine impurities is 100 ppm or less, it became possible to make the VC transparent (see NPL 1).

NPL 2 describes the investigation of radiation-induced polymerization of vinylene carbonate having 99.97 % purity

CITATION LIST

PATENT LITERATURE

PTL 1: JP 2002-8721 A
PTL 2: JP 2002-322171 A
PTL 3: JP 2008-540467 A
PTL 4: JP 2009-29814 A
PTL 5: JP 2010-282760 A
PTL 6: DE102005021965 (A1)
PTL 7: US3457279 (A)
PTL 8: CN1789259 (A)

NON-PATENT LITERATURE

NPL 1: Electrolytes for Lithium and Lithium-Ion Batteries (Modern Aspects of Electrochemistry), Volume 58, 2014, pp 172-173
NPL 2: Radiation polymerization of vinylene carbonate, Schnautz, N. G. et al., ", Report, PER-11", May 1978, pages 11 - 6

## SUMMARY OF INVENTION

### TECHNICAL PROBLEM

The high-purity VC of PTL 1 is VC that is extremely less in impurities. Nevertheless, according to the Wickbold combustion-ion chromatography method, the content of chlorine impurities is not zero.

According to the production method of high-purity VC of PTL 2, the production must be carried out by crystallization with a mixed solvent of toluene and hexane and further distillation, and thus, it is a batch type. Moreover, it may not be said that the production method of PTL 2 is useful as a method of reducing the content of chlorine impurities to substantially zero, and it has become clear that the production method of PTL 2 is not always said to be industrially an efficient method.

In addition, according to the purification method of PTL 3, since the VC is exposed to high heat for a long period of time, during this, a reaction of a minute amount of chlorine, hydrogen chloride, etc. with VC occurs, too, resulting in not only a loss of VC but also formation of chlorine-containing by-products. When the resulting distillate is further purified by the static melt crystallizer, not only it is necessary to perform discontinuous switching of the operation between attachment and sweating of crystals, and no counter-current contact effect is brought. Thus, it has become clear that the purification method of PTL 3 is a purification method in which the impurities are hardly removed.

In the working examples of PTL 4, though VC having a total chlorine amount of 14 ppm is described, VC having a total chlorine amount of less than 14 ppm is not specifically corroborated.

In PTL 5, a nonaqueous electrolytic solution containing VC having a content of a specified chlorine compound of 10 ppm or less is described.

In the working examples of PTL 5, the detection of an ether group containing a chlorine atom, which is represented by a specified general formula (1), is performed by gas chromatography. For that reason, the content of chlorine impurities cannot be quantitatively determined unless a detection peak of each of the ether compounds is specified. Although PTL 5 describes that the content of chlorine-containing vinyl ether-based impurities was reduced, it does not describe at all the reduction of the content of other chlorine impurities that are not a vinyl ether-based material. If only a distillation operation described in PTL 5 is performed, it is impossible to remove all of various chlorine-substituted compounds and a chlorine ion. Moreover, according to the gas chromatography, only a part of chlorine impurities can be detected. From the foregoing matters, it may be considered that when the chlorine impurities of vinylene carbonate obtained in the working examples of PTL 1 are measured by the Wickbold combustion-ion chromatography method, the content of the chlorine impurities becomes about several 10 ppm.

In the light of the above, in the aforementioned PTLs 1 to 5, it is merely disclosed that the content of chlorine impurities as the impurities is reduced to a low concentration, and VC in which the content of chlorine impurities is highly reduced to zero has not been known. As a matter of course, these PTLs 1 to 5 neither describe nor suggest their battery performances, and thus, there was no way to know the battery performances of VC having the content of chlorine impurities of zero.

A problem of the present invention is to provide a high-purity VC in which the content of chlorine impurities is reduced to zero, in other words, a high-purity VC that does not substantially contain impurities at all, a nonaqueous electrolytic solution containing the foregoing high-purity VC, and an energy storage device using the same.

### SOLUTION TO PROBLEM

In order to solve the aforementioned problem, the present inventors made extensive and intensive investigations. As a result, they have successfully reduced the content of chlorine impurities in VC to zero and found that a special phenomenon takes place thereby.

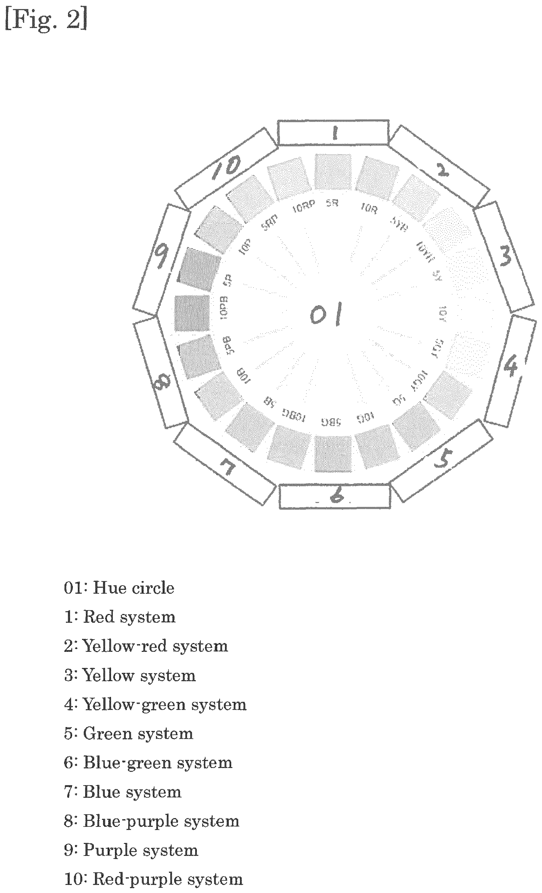

The present inventors have found that as a simple and easy industrial method of reducing the content of chlorine impurities in VC ultimately, such VC can be produced on an industrial scale through crystallization accompanied by solid-liquid counter-current contact, leading to accomplishment of the present invention. The high-purity VC obtained by the foregoing production method, in which the content of chlorine impurities is reduced to zero is a colorless, transparent liquid in a nitrogen atmosphere similar to a conventional VC containing chlorine impurities in an amount of about 100 ppm or less and 10 ppm or more; however, the present inventors have found a peculiar phenomenon in which when exposed in air, the high-purity VC takes on a yellow-green color in terms of the Munsell color system (see FIG. 2).

In the production method of an electrolytic solution for lithium ion batteries, which is classified into Class I petroleums or Class II petroleums, there is a concern of ignition or contamination by water. Therefore, in general, commonsensically, such an electrolytic solution is not exposed in an oxygen-containing atmosphere (for example, in air, etc.), so that it was difficult to discover this phenomenon. Moreover, in a conventional VC containing chlorine impurities in an amount of about 100 ppm or less and 10 ppm or more, even when exposed in an oxygen-containing atmosphere, the foregoing VC was still colorless and transparent and did not take on a yellow-green color.

The present inventors have found that in an energy storage device, such as a lithium secondary battery using a nonaqueous electrolytic solution containing a high-purity VC that when exposed in this oxygen-containing atmosphere, takes on a yellow-green color, etc., an output property at a low temperature and a cycle property over an extremely long period of time are improved.

Specifically, the present invention provides the following [1] to [13].

[1] A high-purity vinylene carbonate (hereinafter also referred to as "high-purity VC") that is VC having the content of chlorine impurities of zero as detected by the Wickbold combustion-ion chromatography method, having coordinated to oxygen, and having a Hazen unit color number (hereinafter referred to as "APHA") in a nitrogen atmosphere of 10 or less, and which, when stored at 45 °C for 7 days in an oxygen-containing atmosphere, takes on a yellow-green color in a range of from 10Y to 10GY in terms of a hue circle of the Munsell color system.

[2] The high-purity VC as set forth above in [1], wherein when stored at 45°C for 7 days in an oxygen-containing atmosphere, the VC has an APHA of 100 or more.

[3] (deleted)

[4] The high-purity VC as set forth above in [1], having a melting point at atmospheric pressure of 20°C or higher and lower than 22°C.

[5] The high-purity VC as set forth above in [1], wherein the chlorine impurities as detected by the Wickbold combustion-ion chromatography method are calculated as converted into a chlorine atom in a manner that a sample is dissolved in a solvent and subjected to oxyhydrogen flame combustion treatment, the obtained gas is absorbed in a sodium carbonate aqueous solution, and the chorine ion in the absorption solution is measured by ion chromatography.

[6] A method for producing the high-purity VC as set forth above in [1], wherein the vinylene carbonate is produced by a method including the following steps (A) to (C):

(A) a step of scraping crude VC crystals crystallized in a crystallization tank by using a scraper and precipitating the VC crystals in a bottom of a melt purification tower;
(B) a step of bringing the precipitated VC crystals and a part of the VC molten liquid melted in the bottom of the melt purification tower into counter-current contact with each other; and
(C) a step of extracting a part of the VC molten liquid from the bottom of the melt purification tower.

[7] A nonaqueous electrolytic solution having an electrolyte salt dissolved in a nonaqueous solvent, the nonaqueous electrolytic solution comprising a high-purity vinylene carbonate, wherein the high-purity vinylene carbonate is a vinylene carbonate having the content of chlorine impurities of zero as detected by the Wickbold combustion-ion chromatography method, having coordinated to oxygen, and having a Hazen unit color number in a nitrogen atmosphere of 10 or less, and which, when stored at 45 °C for 7 days in an oxygen-containing atmosphere, takes on a yellow-green color in a range of from 10Y to 10GY in terms of a hue circle of the Munsell color system.

[8] The nonaqueous electrolytic solution as set forth above in [7], comprising 0.1 to 1.5 mass% of $LiPO_2F_2$ in the nonaqueous electrolytic solution.

[9] The nonaqueous electrolytic solution as set forth above in [7], comprising 1 to 50 ppm of HF in the nonaqueous electrolytic solution.

[10] The nonaqueous electrolytic solution as set forth above in [7], wherein a ratio in concentration between $LiPO_2F_2$ and HF ((HF concentration)/($LiPO_2F_2$ amount) in the nonaqueous electrolytic solution is 1/15,000 to 1/20.

[11] An energy storage device including a positive electrode, a negative electrode, and a nonaqueous electrolytic solution having an electrolyte salt dissolved in a nonaqueous solvent, wherein the nonaqueous electrolytic solution is the nonaqueous electrolytic solution as set forth above in any of [7] to [10].

[12] A method for producing the high-purity VC as set forth above in [1] to [5], comprising bringing crude VC crystals containing chlorine impurities and a part of a molten liquid of the crude VC into solid-liquid counter-current contact with each other.

[13] The method for producing the high-purity VC as set forth above in [12], comprising the following steps (A) to (C):

(A) a step of scraping crude VC crystals crystallized in a crystallization tank by using a scraper and precipitating the VC crystals in a bottom of a melt purification tower;
(B) a step of bringing the precipitated VC crystals and a part of the VC molten liquid melted in the bottom of the melt purification tower into counter-current contact with each other; and
(C) a step of extracting a part of the VC molten liquid from the bottom of the melt purification tower.

ADVANTAGEOUS EFFECTS OF INVENTION

In accordance with the present invention, it is possible to provide a high-purity VC that is entirely free from chlorine impurities as defected by the Wickbold combustion-ion chromatography method, a nonaqueous electrolytic solution containing the foregoing high-purity VC, and an energy storage device using the same.

The energy storage device using the nonaqueous electrolytic solution containing a high-purity VC of the present invention is able to improve an output property at a low temperature and a cycle property over a long period of time. In particular, with respect to the cycle property, a high discharge capacity can be maintained over an extremely long period of time, thereby enabling a life of the energy storage device to be significantly extended, and therefore, the energy storage device of the present invention has an energy saving effect.

As for the evaluation of the cycle property, though the evaluation has hitherto been performed in terms of cycles of about 50 to 100 cycles, the energy storage device using the nonaqueous electrolytic solution containing a high-purity VC of the present invention exhibits an excellent effect even in the evaluation of a cycle property over an extremely long period of time as 1,000 cycles, the evaluation of which has not hitherto been performed.

BRIEF DESCRIPTION OF DRAWINGS

FIG. 1 is a view showing an example of an apparatus for carrying out a production method of a high-purity VC of the present invention.
FIG. 2 is a diagram of a hue circle of the Munsell color system.

DESCRIPTION OF EMBODIMENTS

[High-purity vinylene carbonate]

The high-purity VC of the present invention is vinylene carbonate, in which the content of chlorine impurities (meaning chlorine atom-containing organic compounds or inorganic compounds) as detected by the Wickbold combustion-ion chromatography method is zero, and has a characteristic feature that the vinylene carbonate has a Hazen unit color number in a nitrogen atmosphere of 10 or less.

Different from the conventionally known VC, the high-purity VC of the present invention is VC that does not substantially contain chlorine impurities at all and is a high-purity VC which has not hitherto existed and which can be first obtained by purifying a conventional VC containing chlorine impurities by a method as mentioned later.

According to the Wickbold combustion-ion chromatography method, the measurement is performed in a way that a VC sample is subjected to oxyhydrogen flame combustion treatment, an obtained gas is absorbed in a sodium carbonate aqueous solution, and a chorine ion in the absorption solution is measured by an ion chromatograph, and therefore, all of the chlorine impurities in the VC sample can be surely detected. Moreover, the whole amount of the chlorine impurities is detected as a total chlorine content of $Cl^-$, and therefore, a change of the detection peak of $Cl^-$ in the ion chromatography, or the like is not observed.

In consequence, the terms "the content of chlorine impurities as detected by the Wickbold combustion-ion chromatography method is zero" as referred to in the present specification mean that even if confirmation is performed until a detection limit to be detected by the foregoing method, the presence of a peak cannot be confirmed, namely, even in a measurement limit of the total chlorine content that can be measured at a current technical level, $Cl^-$ cannot be detected.

In the oxyhydrogen flame combustion treatment, for example, the VC sample is dissolved in a solvent, such as an alcohol having 1 to 3 carbon atoms, e.g., methanol, etc., and then subjected to oxyhydrogen flame combustion, followed by absorption in a 5 mM sodium carbonate ($Na_2CO_3$) aqueous solution; however, it should be construed that the foregoing treatment is not limited to this example. Details of the measurement method of the content of chlorine impurities by the Wickbold combustion-ion chromatography method are those as described in the Examples.

The problem of reducing the chlorine content of VC has hitherto been known. However, VC having been highly purified to an extent that "the content of chlorine impurities is zero" has not been known, and actually, it was difficult to product such VC. Furthermore, it cannot be expected at all that VC having been highly purified to an extent that "the content of chlorine impurities is zero" has conspicuously different physical properties as compared with VC that has hitherto been considered to have a high purity.

Moreover, the high-purity VC of the present invention has a characteristic feature that the APHA is 10 or less. Besides, the high-purity VC of the present invention is also characterized by APHA, hue circle, and melting point in the case of storing at 45°C for 7 days in an oxygen-containing atmosphere. Such matters specify the high-purity vinylene carbonate according to the invention of the present application. These matters are hereunder described.

(Hue of high-purity VC)

The high-purity VC of the present invention is colorless and transparent in a nitrogen ($N_2$) atmosphere and has an APHA in a nitrogen atmosphere of 10 or less, and preferably 5 or less.

In the case of storing the high-purity VC of the present invention at 45°C for 7 days in an oxygen ($O_2$)-containing atmosphere, a lower limit of the APHA is 100 or more, more preferably 200 or more, and still more preferably 300 or more.

An upper limit of the APHA in the case of storing the high-purity VC of the present invention at 45°C for 7 days in an oxygen ($O_2$)-containing atmosphere is 500 or less in terms of an APHA of a 35% concentration solution diluted with a solvent that does not disturb the measurement of APHA, such as a carbonate compound, e.g., dimethyl carbonate, diethyl carbonate, etc., and an ester compound, e.g., ethyl acetate, etc., etc., preferably 500 or less in terms of an APHA of a 50% concentration solution diluted with the solvent, and more preferably 500 or less in terms of an APHA in the case of being not diluted with the solvent. When the APHA falls within the aforementioned range, the electrochemical characteristics are improved, and hence, such is preferred.

The measurement of APHA was performed using a color difference meter (ZE6000, manufactured by Nippon Denshoku Industries Co., Ltd.).

Although the high-purity VC of the present invention is colorless and transparent in a nitrogen atmosphere, it develops a color into a yellow-green color in an atmosphere containing oxygen.

The yellow-green color taken on in the case where the high-purity VC of the present invention is stored at 45°C for 7 days in an oxygen-containing atmosphere is a yellow-green system exceeding 5Y but not reaching 5G in the hue circle of the Munsell color system shown in FIG. 2, preferably a yellow-green system ranging from 10Y to 10GY, and more preferably a yellow-green system exceeding 10Y but not reaching 10GY, namely a yellow-green color of GY. A battery using a nonaqueous electrolytic solution containing this high-purity VC is preferred because it improves the output property at a lower temperature and the cycle property over a long period of time.

The aforementioned oxygen-containing atmosphere refers to an atmosphere containing oxygen in an arbitrary concentration, and for example, it may be only oxygen or an atmosphere of oxygen arbitrarily diluted with other gas, and preferably an air atmosphere. It is preferred that the aforementioned atmosphere is a dry atmosphere.

Although the time for storing the high-purity VC of the present invention in an oxygen-containing atmosphere may be an instant, it is preferably 10 minutes or more, and more preferably 30 minutes or more.

The color development of a yellow-green color in the aforementioned oxygen-containing atmosphere is presumed to be resulted from the fact that the high-purity VC that does not substantially contain chlorine impurities at all coordinates to oxygen in air. When the nonaqueous electrolytic solution containing this high-purity VC, in which the color development of a yellow-green color has occurred, is used for an energy storage device, such as a lithium secondary battery, etc., a solid electrolyte interphase (SEI) surface film containing a lot of oxygen is formed on a negative electrode, as compared with a nonaqueous electrolytic solution using a conventionally known VC. Thus, it may be conjectured that in particular, the diffusion of an Li ion in SEI at a low temperature becomes smooth, whereby a low-temperature output property and a cycle property over an extremely long period of time are improved.

When the high-purity VC that does not contain chlorine impurities at all is brought into contact with oxygen in advance to allow the color development of a yellow-green color to occur, higher effects are obtained. However, even in the case where such a high-purity VC is not brought into contact with oxygen but used for a battery in a nitrogen atmosphere as it is, it may be considered that the high-purity VC comes into contact with an extremely small amount of adsorbed oxygen on the electrode surface, which is, however, existent in a considerable extent within the battery, whereby the color development of a yellow-green color occurs within the battery, and thus, the same effects as those mentioned above are obtained.

(Purity and melting point of high-purity VC)

The high-purity VC of the present invention has a purity of 100 mass% and a melting point (at atmospheric pressure) of 20°C or more and lower than 22°C, and it belongs to the range of designated flammable goods (flammable solids) in the classification of dangerous goods of the Fire Service Law.

However, in the conventional VC that is colorless and transparent or takes on a yellow to brown color in an oxygen-containing atmosphere (it is described on page 2604 of Aldrich Reagent Catalog, 2012-2014 that the melting point is 19°C to 22°C), the melting point (at atmospheric pressure) is lower by 1°C to 2°C than the high-purity VC of the present invention and is 19°C or higher and lower than 20°C. Thus, different from the high-purity VC of the present invention, the conventional VC belongs to the range of Dangerous Material Class 4, Class III petroleums. As for the reason for this, it may be presumed that in the high-purity VC of the present invention, by reducing the content of chlorine impurities to substantially zero, thereby enabling the purity to reach 100 mass%, the melting point increases. This difference in the melting point is also one of evidences supporting the special phenomenon.

A lower limit of the melting point which the high-purity VC of the present invention exhibits is 20°C or higher, and preferably 20.5°C or higher at atmospheric pressure. An upper limit of the melting point is lower than 22°C.

Here, the melting point as referred to in the specification of the present application is defined in terms of a temperature at which the solid VC has been completely dissolved.

When the high-purity VC having a melting point falling within the aforementioned range is used as an additive of a nonaqueous electrolytic solution for energy storage device, it exhibits more excellent effects for improving the output property and so on than the conventional VC.

[Production method of high-purity vinylene carbonate]

It is preferred that the high-purity VC of the present invention is produced by a combined method of a crystallization method and a counter-current contact method. That is, the production method of the high-purity VC of the present invention is a method including bringing crude VC crystals containing chlorine impurities (general VC crystals containing chlorine impurities after purification) and a part of a molten liquid of the crude VC into solid-liquid counter-current contact with each other.

Specifically, a method including the following steps (A) to (C) is preferred as the production method of the high-purity VC of the present invention.

(A) A step of scraping crude VC crystals crystallized in a crystallization tank by using a scraper and precipitating the VC crystals in a bottom of a melt purification tower.
(B) A step of bringing the precipitated VC crystals and a part of the VC molten liquid melted in the bottom of the melt purification tower into counter-current contact with each other.
(C) A step of extracting a part of the VC molten liquid from the bottom of the melt purification tower.

In the aforementioned production method of the high-purity VC of the present invention, the high purity can be attained by performing the melt purification including the steps (A) to (C) only one time. In view of the fact that the VC has a double bond, it is likely subjected to denaturation by thermal history. Thus, though it is desired that the thermal history is minimized as far as possible, the present invention is also advantageous from this point with respect to the method of the present invention.

On the other hand, it is described in paragraph [0016] of PTL 5 that the purification operation is performed two or more times. Taking into consideration the matter that in the conventional method, even when the purification operation is performed two or more times, the VC cannot be thoroughly purified, superiority of the production method of the high-purity VC of the present invention is evident.

The crude VC that is used for obtaining the high-purity VC of the present invention is one obtained by the conventionally known production method. For the purpose of removing impurities to some extent in advance, the crude VC may be purified by distillation and is no means limited with respect to species of impurities, production method, and so on.

In the production method of the high-purity VC of the present invention, by crystallizing the crude VC and then purifying the resulting crystals by the counter-current contact method, VC that is purified in a high purity such that the content of chlorine impurities is substantially zero can be obtained. The counter-current contact method is a method in which a purification effect is produced due to the contact of the crude VC crystals with the crude VC molten liquid, whereby the high-purity VC having been purified to such an extent that the content of chlorine impurities is zero can be obtained.

As a preferred specific example of a purification apparatus that is used for the counter-current contact method, there is exemplified an apparatus configured of a crystallization tank and a melt purification tower and provided with a heating unit in a bottom of the melt purification tower.

The crude VC crystals that are treated by such a purification apparatus can be supplied into the apparatus by an arbitrary method. For example, there are exemplified a method of supplying a slurry containing the crude VC crystals directly into the melt purification tower; and a method of supplying a VC-containing solution or molten liquid into the crystallization tank, crystallizing it, and then scraping the crystals. Of those, according to the method of supplying the crude VC molten liquid into the crystallization tank, crystallizing the crude VC, and then scraping and falling the crystals, the crude VC crystals are efficiently supplied into the melt purification tower, and there is no concern of occurrence of clogging, and hence, such a method preferred.

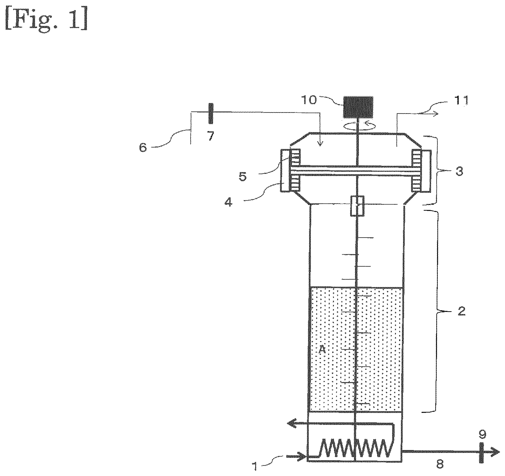

As one example of such a method, there is exemplified a method of using a vertical melt purification tower configured as shown in FIG. 1. According to this method, the crude VC crystals are precipitated from an upper part of the melt purification tower and brought into counter-current contact with a part of the VC molten liquid melted in a bottom of the melt purification tower, thereby accumulating the VC crystals in the bottom of the melt purification tower while more increasing the purity. By extracting a part of the resulting molten liquid from the bottom of the melt purification tower, the high-purity VC can be obtained.

Next, the production apparatus of the high-purity VC, which is suitably used in the present invention, is more specifically described.

(Production apparatus of high-purity VC)

An apparatus for bringing the crude VC crystals and a part of the crude VC molten liquid into solid-liquid counter-current contact with each other to achieve the purification is preferred as the production apparatus of high-purity VC that is used in the present invention.

FIG. 1 is a view showing an example of a crystallization-melt purification apparatus for carrying out the production method of the present invention. This apparatus is a vertical apparatus including a heating part 1 in a bottom thereof, a melt purification tower 2 in a middle part thereof, and a crystallization tank 3 having a scraping-type indirect cooling crystallization unit in a upper part thereof, and the crystallization tank 3 and the melt purification tower 2 are connected directly with each other and continued within the apparatus.

In FIG. 1, a crude VC molten liquid (raw material) is introduced into the crystallization tank 3 from a crude VC molten liquid supply pipe 6 through a raw material filter 7 and subjected to melt purification.

The apparatus is configured in such a manner that a cooling unit 4, such as a cooling jacket, etc., is provided on the periphery of the crystallization tank 3, a coolant is supplied into this cooling unit 4 to cool indirectly the wall surface of the crystallization tank 3, thereby rendering the crude VC molten liquid in a supersaturated state, and the crude VC is crystallized. In the crystallization tank 3, a scraper 5 including plural scraping blades (not shown) to be rotatably driven by a driving unit is provided in a height direction. The crude VC crystals deposited on the inner wall surface of the crystallization tank 3 are scraped by the scraper 5, and the scraped crude VC crystals fall within the melt purification tower 2 and are further precipitated in the bottom of the melt purification tower 2.

Meanwhile, a part of the molten liquid of the crystallization tank 3 is extracted as a mother liquor from a mother liquor extraction pipe 11, and this mother liquor is again supplied into the crystallization tank 3 through the supply pipe 6.

From the viewpoints of scraping operability of the crude VC crystals and so on, a pressure (evaporation pressure) of the crystallization tank 3 is preferably around an atmospheric pressure, and desirably 4 atm or less.

The melt purification tower 2 includes a stirrer 10 and includes the heating part 1 provided with a melt heating unit, such as a heating heater, etc., in the bottom thereof. The stirrer 10 stabilizes the behavior of crystal particles in a molten layer in a lower region of the melt purification tower 2 and is provided with a horizontal stirring blade installed on a stirring shaft that is made rotatable by a driving motor.

In the melt purification tower 2, the crude VC crystals are heat melted by the heating part 1 to generate an upward flow as a reflux liquid, thereby ascending it in the group of the crude VC crystals scraped from the crystallization tank 3. The crystals scraped from the crystallization tank 3 come into solid-liquid counter-current contact with the upward flow of the high-purity VC molten liquid melted in the bottom of the melt purification tower 2; the crystal surfaces having a high content of chlorine impurities are simultaneously melt cleaned while being dispersed; and the VC crystals are precipitated and accumulated in the bottom of the melt purification tower 2 while increasing the purity thereof, thereby forming an accumulation layer (A). In this melt purification tower 2, the crude VC crystals and the molten liquid are surely subjected to solid-liquid counter-current contact with each other, and therefore, the purification efficiency is extremely high.

The high-purity VC as a product is taken out as an extraction liquid from a product extracting pipe 8 in the bottom of the melt purification tower 2 through a product filter 9 while separating a crystal. A molten material other than the extraction liquid ascends as a reflux liquid within the melt purification tower 2. Such operations are continuously performed, whereby the purification effect of crude VC can be much more enhanced.

A heating temperature in the bottom of the melt purification tower 2 is not particularly limited so long as it is the melting point of VC to be extracted from the product extracting pipe 8 or higher. However, from the viewpoint of suppressing thermal degradation of the high-purity VC as a product, the heating temperature is preferably lower. In consequence, the heating temperature in the bottom of the melt purification tower 2 is typically 20 to 35°C, preferably 21 to 30°C, and more preferably 21 to 25°C.

[Nonaqueous electrolytic solution]

The nonaqueous electrolytic solution of the present invention can be obtained by dissolving a generally known electrolyte salt in a generally known nonaqueous solvent and adding the high-purity VC of the present invention thereto. Although the addition amount of the VC is arbitrary, it is preferably 0.01 mass% or more, more preferably 0.05 mass% or more, still more preferably 0.1 mass% or more, and especially preferably 0.2 mass% or more, and preferably 20 mass% or less, more preferably 15 mass% or less, still more preferably 10 mass% or less, and especially preferably 5 mass% or less in the nonaqueous electrolytic solution. That is, the addition amount of the VC is preferably 0.01 to 20 mass%, more preferably 0.05 to 15 mass%, still more preferably 0.1 to 10 mass%, and especially preferably 0.2 to 5 mass%.

On that occasion, the nonaqueous solvent used is preferably one resulting from performing purification in advance to minimize impurities as far as possible within the range where the productivity is not conspicuously lowered.

(Nonaqueous solvent)

Examples of the nonaqueous solvent that is used for the nonaqueous electrolytic solution of the present invention include a cyclic carbonate, a linear carbonate, a linear carboxylic acid ester, a lactone, an ether, an amide, a nitrile, a phosphoric acid ester, an S=O bond-containing compound, a carboxylic acid anhydride, an aromatic compound, and the like.

From the viewpoint of more effectively exhibiting the effects of the present invention, it is preferred that the nonaqueous solvent contains a cyclic carbonate, and it is more preferred that the nonaqueous solvent contains a cyclic carbonate and a linear carbonate.

As the cyclic carbonate, one or more selected from ethylene carbonate (EC), propylene carbonate (PC), 4-fluoro-1,3-dioxolan-2-one (FEC), trans- or cis-4,5-difluoro-1,3-dioxolan-2-one (the both will be hereunder named generically as "DFEC"), and vinyl ethylene carbonate (VEC) are suitably exemplified. Of those, one or more selected from, in addition to EC and PC, combinations, such as EC and FEC, EC and VEC, EC and PC, PC and DFEC, EC, FEC and PC, and the like are preferred.

Although the content of the cyclic carbonate is not particularly limited, it is preferred to use the cyclic carbonate in an amount ranging from 0 to 40 volume% of the total volume of the nonaqueous solvent. When the foregoing content is more than 40 volume%, there is a case where the viscosity of the nonaqueous electrolytic solution increases, and hence, it is preferred that the content falls within the aforementioned range.

Examples of the linear carbonate include one or more asymmetric linear carbonates selected from methyl ethyl carbonate (MEC), methyl propyl carbonate, methyl isopropyl carbonate, methyl butyl carbonate, and ethyl propyl carbonate; and one or more symmetric linear carbonates selected from dimethyl carbonate (DMC), diethyl carbonate (DEC), dipropyl carbonate, and dibutyl carbonate.

In particular, when an asymmetric linear carbonate is contained, battery characteristics, such as a long-term cycle property, etc., tend to be improved, and hence, such is preferred. It is also preferred to use an asymmetric linear carbonate and a symmetric linear carbonate in combination. MEC is more preferred as the asymmetric linear carbonate, and DMC is more preferred as the symmetric linear carbonate.

Although the content of the linear carbonate is not particularly limited, it is preferred to use the linear carbonate in an amount ranging from 60 to 100 volume% of the total volume of the nonaqueous solvent. When the foregoing content is less than 60 volume%, there is a case where the viscosity of the nonaqueous electrolytic solution increases, and hence, it is preferred that the content falls within the aforementioned range.

From the viewpoint of improving the battery characteristics, such as a long-term cycle property, etc., a proportion of the cyclic carbonate and the linear carbonate is preferably 10/90 to 40/60, more preferably 15/85 to 35/65, and still more preferably 20/80 to 30/70 in terms of a (cyclic carbonate)/(linear carbonate) volume ratio.

(Electrolyte salt)

As the electrolyte salt that is used in the present invention, there are suitably exemplified a lithium salt and an onium salt composed of a combination of onium cation and anion.

As the lithium salt, one or more selected from inorganic lithium salts, such as $LiPF_6$, $LiPO_2F_2$, $Li_zPO_3F$, $LiBF_4$, $LiClO_4$, etc.; and lithium salts containing a linear fluorinated alkyl group, such as $LiN(SO_2CF_3)_2$, $LiN(SO_2C_2F_5)_2$, etc., are preferred; and one or more selected from $LiPF_6$, $LiPO_2F_2$, and $LiBF_4$ are more preferred. It is still more preferred that $LiPF_6$ and a small amount of $LiPO_2F_2$ are contained.

A concentration of the electrolyte salt in the nonaqueous electrolytic solution is preferably 0.3 M or more, more preferably 0.5 M or more, and still more preferably 0.8 M or more, and preferably 4 M or less, more preferably 3 M or less, and still more preferably 2 M or less.

It has become clear that by mixing the high-purity VC of the present invention with an electrolytic solution containing an electrolyte salt preferably containing 0.1 mass% or more and 2 mass% or less of lithium difluorophosphate ($LiPO_2F_2$) in the nonaqueous electrolytic solution, an effect for further improving the low-temperature output property is brought. Although the reason for this is not always elucidated yet, it may be considered that the VC having coordinated to oxygen assists the effect of $LiPO_2F_2$ in the electrolytic solution. Specifically, it may be presumed that when $LiPO_2F_2$ that improves the output due to an interaction with the electrode and the VC having coordinated to oxygen undergo an interaction on the electrode, a surface film containing a lot of an oxygen atom assisting ionic conduction is formed on the surface of an electrode active material, resulting in improving the low-temperature output.

Although the content of $LiPO_2F_2$ that is contained in the nonaqueous electrolytic solution of the present invention is not particularly limited, a lower limit of the content of $LiPO_2F_2$ is preferably 0.1 mass% or more, more preferably 0.2 mass% or more, still more preferably 0.3 mass% or more, especially preferably 0.6 mass% or more, and most preferably 0.8 mass% or more in the nonaqueous electrolytic solution. When the content of $LiPO_2F_2$ is the aforementioned lower limit value or more, the effect for improving the output at a lower temperature is enhanced, and hence, such is preferred. An upper limit of the content of $LiPO_2F_2$ is preferably 1.5 mass% or less, more preferably 1.3 mass% or less, still more preferably 1.0 mass% or less, and yet still more preferably 0.8 mass% or less. When the content of $LiPO_2F_2$ is the aforementioned upper limit value or less, the effect for improving the output at a lower temperature is enhanced, and hence, such is preferred.

A lower limit of the concentration of HF that is contained in the nonaqueous electrolytic solution of the present invention is preferably 1 ppm or more, and more preferably 2 ppm or more. When the HF concentration is the aforementioned lower limit value or more, the effect for improving the output at a lower temperature is enhanced, and hence, such is preferred. When an upper limit of the HF concentration is preferably 50 ppm or less, more preferably 20 ppm or less, and still more preferably 8 ppm or less, the effect for improving the output at a lower temperature is enhanced, and hence, such is preferred.

A ratio of the HF concentration to the $LiPO_2F_2$ content (((HF concentration)/($LiPO_2F_2$ content)) (this ratio is a ratio in the case of expressing both the content of $LiPO_2F_2$ and the HF concentration in terms of ppm) is preferably 1/15,000 to 1/20, and more preferably 1/10,000 to 1/20. An upper limit of the aforementioned ratio is preferably 1/220 or less, and more preferably 1/500 or less.

When the ratio of ((HF concentration)/($LiPO_2F_2$ content)) falls within the aforementioned range, an influence of HF that impairs the interaction between $LiPO_2F_2$ and VC having coordinated to oxygen is suppressed, and such is more preferred.

[Energy storage device]

The energy storage device of the present invention is an energy storage device including a positive electrode, a negative electrode, and a nonaqueous electrolytic solution having an electrolyte salt dissolved in a nonaqueous solvent, wherein the nonaqueous electrolytic solution is the nonaqueous electrolytic solution of the present invention.

Examples of the energy storage device of the present invention include a lithium primary or secondary battery using a lithium salt for the electrolyte salt, a lithium ion capacitor (an energy storage device of storing energy by utilizing intercalation of a lithium ion into a carbon material, such as graphite that is a negative electrode, etc.), an electric double layer capacitor (an energy storage device of storing energy by utilizing an electric double layer capacitance in an interface between the electrolytic solution and the electrode), and the like. Of those, a lithium battery is preferred, a lithium secondary battery is more preferred, and a lithium ion secondary battery is most suitable.

The energy storage device of the present invention can be, for example, obtained by including generally used positive electrode and negative electrode, and the aforementioned nonaqueous electrolytic solution.

As a positive electrode active material of an energy storage device, particularly a lithium secondary battery, a complex metal oxide with lithium containing one or more selected from cobalt, manganese, and nickel; and a lithium-containing olivine-type phosphate including one or more selected from iron, cobalt, nickel, and manganese are preferred.

As a negative electrode active material of an energy storage device, particularly a lithium secondary battery, there are exemplified a metal lithium, a lithium alloy, a carbon material capable of absorbing and releasing lithium, tin (elemental substance), a tin compound, silicon (elemental substance), a silicon compound, a lithium titanate compound, and the like. In the ability of absorbing and releasing a lithium ion, a high-crystalline carbon material, such as artificial graphite, natural graphite, etc., is preferred, and a carbon material having a graphite-type crystal structure is more preferred.

EXAMPLES

The present invention is hereunder described in more detail by reference to Examples, but it should not be construed that the present invention is limited to the following Examples so long as the gist thereof is not deviated. The content of chlorine impurities and APHA in VC were measured by the following methods.

(1) Measurement of the content of chlorine impurities in VC by the Wickbold combustion-ion chromatography method:

2.0 g of a VC sample was dissolved in 2 mL of methanol and subjected to oxyhydrogen flame combustion treatment using an oxyhydrogen flame composition apparatus (a trade name: TSN-LS Model, manufactured by Toka Seiki Co., Ltd.); the obtained gas was absorbed in a 5.0 mM $Na_2CO_3$ aqueous solution; a chlorine ion in the absorption solution was measured using an ion chromatograph (a trade name: DX-320, manufactured by Thermo Fisher Scientific K.K. (old Nippon Dionex K.K.) under the following conditions; and the content of chlorine impurities was calculated as converted into a chlorine atom.

- • Column: Guard column, IonPac AG12A, Dionex
- • Separation column: IonPac AS12A, Dionex
- • Eluant: 2.7 mM sodium carbonate-0.3 mM sodium hydrogencarbonate
- • flow rate: 1.5 mL/min
- • Suppressor: AMMS anion membrane suppressor
- • Regenerant: 12.5 mM sulfuric acid solution
- • Detector: Electroconductivity detector

(2) Measurement of APHA:

The measurement of APHA of VC was performed using a color difference meter (ZE6000, manufactured by Nippon Denshoku Industries Co., Ltd.).

(3) Measurement of melting point:

The measurement of the melting point of VC was performed by a visual inspection method as described in JIS K0064.

[Synthesis Example 1]

An apparatus the same as in FIG. 1 was used as a crystallization-melt purification apparatus. The melt purification tower 2 has an inner diameter of 220 mm and an effective height (a height from a melting part in a lower part of the tower to a tower top) of 2,000 mm, and the crystallization tank 3 has an outer diameter of 350 mm and an effective height of 850 mm, and includes the scraper 5 in the interior thereof. Cold water (inlet: 11.2°C, outlet: 11.7°C) was passed through the cooling unit 4. Warm water (heating inlet: 30.0°C, heating outlet: 27.9°C) was passed as a heating medium through the heating part 1. A stirring shaft having horizontal stirring blades was used as the stirrer 10. A membrane cartridge filter (filter medium: PTFE) having a pore diameter of 1.0 $\mu$m was installed in each of the crude VC supply pipe 6 and the product extracting pipe 8.

A crude VC molten liquid (VC amount: 99.20 mass%, content of chlorine impurities: 2,789 ppm) was supplied from the crude VC supply pipe 6.

Crude VC crystals are deposited on the inner wall of the crystallization tank 3, and crude VC crystals scraped by the scraper 5 are precipitated in a bottom of the melt purification tower 2. When the crude VC crystals were brought into solid-liquid counter-current contact with a part of the VC molten liquid melted in the bottom of the melt purification tower 2, the crude VC crystals were precipitated in the bottom of the tower while increasing the purity thereof, thereby forming the accumulation layer (A). At this point of time, the operation was continued for 24 hours while controlling the temperature of each of the warm water and the cold water without extracting the VC molten liquid from the product extracting pipe 8, until the temperature distribution within the melt purification tower 2 became stable. Thereafter, the extraction of the VC molten liquid from the product extracting pipe 8 was commenced.

The operation was further continued for 2 hours. After the matter that the accumulation layer of the crystals became free from disturbance was confirmed through visual inspection and the temperature distribution of the melt purification tower 2, the high-purity VC was extracted from the product extracting pipe 8. As a result, the content of chlorine impurities in the purified VC was zero. In addition, a proportion of the high-purity VC obtained from the product extracting pipe 8 to the amount of the crude VC supplied from the crude VC supply pipe 6 was 0.95.

$$\text{(Amount of obtained high-purity VC (kg))/(Amount of supplied crude VC (kg))} = 0.95$$

[Storage test of VC]

With respect to the high-purity VC (Compound 1) obtained by the method of Synthesis Example 1, immediately after purification and after storage at 45°C for 7 days in a nitrogen atmosphere or in an oxygen-containing atmosphere (in dry air), the APHA of VC was measured without being diluted. In addition, Compound 2 and Compound 3 each containing chlorine impurities were stored in the same manner as in Compound 1 and measured for the APHA. The results are shown in Table 1.

Table 1

| | Content of chlorine impurities (ppm) | APHA (hue) | | | Melting point (°C) |
|---|---|---|---|---|---|
| | | Nitrogen atmosphere | | Oxygen-containing atmosphere | |
| | | Immediately after purification | After storage at 45°C for 7 days | After storage at 45°C for 7 days | |
| Compound 1 | None | <10 (colorless) | <10 (colorless) | 300 (5GY) | 20.6 to 20.9 |
| Compound 2 | 14 | <10 (colorless) | <10 (colorless) | <10 (colorless) | 20.0 to 20.4 |
| Compound 3 | 305 | <10 (colorless) | 150 (5YR) | 150 (5YR) | 19.2 to 19.8 |

[Production of lithium ion secondary battery]

94 mass% of $LiNi_{1/3}Mn_{1/3}Co_{1/3}O_2$ and 3 mass% of acetylene black (electroconductive agent) were mixed and then added to and mixed with a solution which had been prepared by dissolving 3 mass% of polyvinylidene fluoride (binder) in 1-methyl-2-pyrrolidone in advance, thereby preparing a positive electrode mixture paste. This positive electrode mixture paste was applied onto one surface of an aluminum foil (collector), dried, and treated under pressure, followed by cutting into a predetermined size, thereby producing a positive electrode sheet in a belt-like form.

95 mass % of artificial graphite was added to and mixed with a solution which had been prepared by dissolving 5 mass% of polyvinylidene fluoride (binder) in 1-methyl-2-pyrrolidone in advance, thereby preparing a negative electrode mixture paste. This negative electrode mixture paste was applied onto one surface of a copper foil (collector), dried, and treated under pressure, followed by cutting into a predetermined size, thereby producing a negative electrode sheet.

1.1 mol/L of $LiPF_6$ was dissolved in a mixed solvent of ethylene carbonate (EC), methyl ethyl carbonate (MEC), and dimethyl carbonate (DMC) in a volume ratio of 30/30/40, thereby preparing a nonaqueous electrolytic solution.

Then, VCs before and after the aforementioned storage test were each added in an amount of 2 mass% to the nonaqueous electrolytic solution and used for each of lithium ion secondary batteries of Examples 1 to 6 and Comparative Examples 1 to 4. In Examples 4 to 6 and Comparative Example 4, the amount of $LiPO_2F_2$ resulting from totaling of $LiPO_2F_2$ formed upon hydrolysis of $LiPF_6$ in the electrolytic solution and $LiPO_2F_2$ intentionally added was regulated in an amount shown in Table 2. The concentration of HF contained in the nonaqueous electrolytic solution of each of Examples 4 to 6 and Comparative Example 4 was regulated to 21 ppm ((HF concentration)/($LiPO_2F_2$ content) $\cong$ 1/238) in Example 4, 8 ppm ((HF concentration)/($LiPO_2F_2$ content) $\cong$ 1/625) in Example 5, and 14 ppm ((HF concentration)/($LiPO_2F_2$ content) $\cong$ 1/857) in Example 6, respectively.

The positive electrode sheet, a separator, and the negative electrode sheet were laminated in this order, and the aforementioned nonaqueous electrolytic solution was added, thereby producing a laminate-type battery, followed by performing evaluations.

[Evaluation of high-temperature cycle property]

<Initial discharge capacity>

In a thermostatic chamber at 25°C, the laminate-type battery produced by the aforementioned method was charged up to a final voltage of 4.2 V with a constant current of 1 C and under a constant voltage for 3 hours and subsequently discharged down to a final voltage of 2.7 V with a constant current of 1C, thereby determining an initial discharge capacity.

<Discharge capacity retention rate after high-temperature cycle>

Subsequently, in a thermostatic chamber at 60°C, this laminate-type battery was treated by repeating a cycle of charging up to a final voltage of 4.2 V with a constant current of 1 C and under a constant voltage for 3 hours and subsequently discharging down to a discharge voltage of 2.7 V with a constant current of 1 C, until it reached 1,000 cycles. Then, a discharge capacity retention rate was determined according to the following equation.

$$\text{Discharge capacity retention rate (\%)} = ((\text{Discharge capacity after 1,000 cycles})/(\text{Discharge capacity after 1st cycle})) \times 100$$

<Impedance after high-temperature cycle>

In a thermostatic chamber at 0°C, the battery after 1,000 cycles was measured for an impedance at a frequency of 1 kHz, thereby evaluating a low-temperature output property. A resistance ratio was determined according to the following expression while defining the impedance of Comparative Example 1 as 100.

$$\text{Resistance ratio (\%)} = ((\text{Impedance})/(\text{Impedance of Comparative Example 1})) \times 100$$

It is meant that as the lower this resistance ratio (%), the more excellent the low-temperature output property is.

The conditions regarding VC and $LiPO_2F_2$ in the electrolytic solution and the results of battery evaluation are shown in Table 2.

Table 2

|  | VC | Regulation of $LiPO_2F_2$ amount | Storage conditions at 45°C for 7 days | Discharge capacity retention rate after 1,000 cycles (%) | Impedance (resistance ratio: %) |
|---|---|---|---|---|---|
| Example 1 | Compound 1 | None | No | 70.4 | 97 |
| Example 2 | Compound 1 | None | Nitrogen atmosphere | 70.2 | 97 |
| Comparative Example 1 | Compound 2 | None | Nitrogen atmosphere | 67.4 | 100 |
| Example 3 | Compound 1 | None | Oxygen-containing atmosphere | 71.8 | 96 |
| Comparative Example 2 | Compound 2 | None | Oxygen-containing atmosphere | 66.8 | 100 |
| Example 4 | Compound 1 | 0.5 wt% | Oxygen-containing atmosphere | 72.0 | 89 |
| Example 5 | Compound 1 | 0.5 wt% | Oxygen-containing atmosphere | 72.4 | 88 |
| Example 6 | Compound 1 | 1.2 wt% | Oxygen-containing atmosphere | 73.4 | 86 |
| Comparative Example 3 | Compound 3 | None | Nitrogen atmosphere | 61.1 | 112 |
| Comparative Example 4 | Compound 3 | 0.5 wt% | Nitrogen atmosphere | 61.0 | 109 |

It is noted that as compared with the lithium ion secondary batteries obtained in Comparative Examples 1 to 4, all of the lithium ion secondary batteries using the nonaqueous electrolytic solution containing the high-purity VC (Compound 1) having the content of chlorine impurities of zero of Examples 1 to 6 are excellent in the cycle property over an extremely long period of time as 1,000 cycles at a high temperature and are also improved in the output property at a low temperature.

More specifically explaining, as compared with the lithium ion secondary batteries using the nonaqueous electrolytic solution containing VC (Compound 2) having the total content of chlorine impurities of 14 ppm of Comparative Examples 1 and 2, in the lithium ion secondary batteries using the nonaqueous electrolytic solution containing the high-purity VC (Compound 1) having the content of chlorine impurities of zero of Examples 2 and 3, the discharge capacity retention rate (%) after 1,000 cycles is improved from 67.4% (Comparative Example 1) to 70.2% (Example 2) and improved from 66.8% (Comparative Example 2) to 71.8% (Example 3), respectively. It is noted from this fact that the overall uptime of an energy storage device, such as a lithium secondary battery, etc., can be largely improved.

In addition, with respect to the output property at a low temperature (resistance ratio (%)), the output property at a low temperature of Examples 2 and 3 is low as 97% and 96%, respectively and is excellent in the output property at a low temperature relative to 100 of Comparative Examples 1 and 2.

Furthermore, as compared with the lithium ion secondary battery using the nonaqueous electrolytic solution containing VC (Compound 3) having the total content of chlorine impurities of 305 ppm and $LiPO_2F_2$ of Comparative Example 4, in the lithium ion secondary batteries using the nonaqueous electrolytic solution containing the high-purity VC (Compound 1) having the content of chlorine impurities of zero and $LiPO_2F_2$ of Examples 4 to 6, the discharge capacity retention rate (%) after 1,000 cycles is improved from 61.0% (Comparative Example 4) to 70.2% (Example 4) to 73.4% (Example 6). These effects are largely improved even in comparison with Examples 1 to 3 not containing $LiPO_2F_2$. It is noted from this fact that the overall uptime of an energy storage device, such as a lithium secondary battery, etc., can be much more improved.

In addition, with respect to the output property at a low temperature (resistance ratio (%)), as compared with Comparative Example 4 (109%), in Examples 4 to 6, it is low as 86% to 89%, so that the output property at a low temperature is much more excellent.

In the light of the above, the present invention exhibits unexpectedly remarkable effects relative to Comparative Examples 1 and 2 corresponding to the conventional technologies.

REFERENCE SIGNS LIST

1: Heating part
2: Melt purification tower
3: Crystallization tank
4: Cooling unit
5: Scraper
6: Crude VC molten supply pipe
7: Raw material filter
8: Product extracting pipe
9: Product filter
10: Stirrer
11: Mother liquor extraction pipe

INDUSTRIAL APPLICABILITY

By using the nonaqueous electrolytic solution containing a high-purity VC of the present invention, an energy storage device that is excellent in electrochemical characteristics, such as an output property at a low temperature, a cycle property over a long period of time, etc., can be obtained. The obtained energy storage device has a long life and an energy saving effect.

**Claims**

1. A nonaqueous electrolytic solution having an electrolyte salt dissolved in a nonaqueous solvent, the nonaqueous electrolytic solution comprising a high-purity vinylene carbonate, wherein the high-purity vinylene carbonate is a vinylene carbonate having the content of chlorine impurities of zero as detected by the Wickbold combustion-ion chromatography method, having coordinated to oxygen, and having a Hazen unit color number in a nitrogen atmosphere of 10 or less, and which, when stored at 45 °C for 7 days in an oxygen-containing atmosphere, takes on a yellow-green color in a range of from 10Y to 10GY in terms of a hue circle of the Munsell color system.

2. The nonaqueous electrolytic solution according to claim 1, comprising 0.1 to 1.5 mass% of lithium difluorophosphate in the nonaqueous electrolytic solution.

3. The nonaqueous electrolytic solution according to claim 1, comprising 1 to 50 ppm of HF in the nonaqueous electrolytic solution.

4. The nonaqueous electrolytic solution according to claim 1, wherein a ratio in concentration between $LiPO_2F_2$ and HF ((HF concentration)/($LiPO_2F_2$ amount)) in the nonaqueous electrolytic solution is 1/15,000 to 1/20.

5. An energy storage device comprising a positive electrode, a negative electrode, and a nonaqueous electrolytic solution having an electrolyte salt dissolved in a nonaqueous solvent, wherein the nonaqueous electrolytic solution is the nonaqueous electrolytic solution according to any of claims 1 to 4.

6. A high-purity vinylene carbonate that is a vinylene carbonate having the content of chlorine impurities of zero as detected by the Wickbold combustion-ion chromatography method, having coordinated to oxygen, and having a Hazen unit color number of 10 or less in a nitrogen atmosphere, and which, when stored at 45 °C for 7 days in an oxygen-containing atmosphere, takes on a yellow-green color in a range of from 10Y to 10GY in terms of a hue circle of the Munsell color system.

7. The high-purity vinylene carbonate according to claim 6, which, when stored at 45°C for 7 days in an oxygen-containing atmosphere, has an APHA of 100 or more.

8. The high-purity vinylene carbonate according to claim 6, having a melting point at atmospheric pressure of 20°C or higher and lower than 22°C.

9. The high-purity vinylene carbonate according to claim 6, wherein the chlorine impurities as detected by the Wickbold combustion-ion chromatography method are calculated as converted into a chlorine atom in a manner that a sample is dissolved in a solvent and subjected to oxyhydrogen flame combustion treatment, the obtained gas is absorbed in a sodium carbonate aqueous solution, and the chorine ion in the absorption solution is measured by ion chromatography.

10. A method for producing the high-purity vinylene carbonate as defined in claim 6, wherein the vinylene carbonate is produced by a method including the following steps (A) to (C):

(A) a step of scraping crude vinylene carbonate crystals crystallized in a crystallization tank by using a scraper and precipitating the vinylene carbonate crystals in a bottom of a melt purification tower;
(B) a step of bringing the precipitated vinylene carbonate crystals and a part of the vinylene carbonate molten liquid melted in the bottom of the melt purification tower into counter-current contact with each other; and
(C) a step of extracting a part of the vinylene carbonate molten liquid from the bottom of the melt purification tower.

11. A method for producing the high-purity vinylene carbonate according to any of claims 6 to 9, comprising bringing crude vinylene carbonate crystals containing chlorine impurities and a part of a molten liquid of the crude vinylene carbonate into solid-liquid counter-current contact with each other.

12. The method for producing the high-purity vinylene carbonate according to claim 11, comprising the following steps (A) to (C):

(A) a step of scraping crude vinylene carbonate crystals crystallized in a crystallization tank by using a scraper and precipitating the vinylene carbonate crystals in a bottom of a melt purification tower;
(B) a step of bringing the precipitated vinylene carbonate crystals and a part of the vinylene carbonate molten liquid melted in the bottom of the melt purification tower into counter-current contact with each other; and
(C) a step of extracting a part of the vinylene carbonate molten liquid from the bottom of the melt purification tower.

**Patentansprüche**

1. Nichtwässrige elektrolytische Lösung mit einem in einem nichtwässrigen Lösemittel gelösten Elektrolytsalz, wobei die nichtwässrige elektrolytische Lösung ein hochreines Vinylencarbonat umfasst, wobei das hochreine Vinylencarbonat ein Vinylencarbonat ist, das den Gehalt an Chlorverunreinigungen von Null aufweist, wie durch das Wickbold-Verbrennungs-Ionenchromatographieverfahren nachgewiesen, das an Sauerstoff koordiniert ist, und in einer Stickstoffatmosphäre eine Hazen-Einheit-Farbzahl von 10 oder weniger aufweist, und das, wenn für 7 Tage bei 45 °C in einer sauerstoffhaltigen Atmosphäre gelagert, eine gelb-grüne Farbe in einem Bereich von 10Y bis 10GY, bezüglich eines Tönungskreises des Munsell-Farbsystems, annimmt.

2. Nichtwässrige elektrolytische Lösung nach Anspruch 1, umfassend 0,1 bis 1,5 Massen-% an Lithiumdifluorphosphat in der nichtwässrigen elektrolytischen Lösung.

3. Nichtwässrige elektrolytische Lösung nach Anspruch 1, umfassend 1 bis 50 ppm an HF in der nichtwässrigen elektrolytischen Lösung.

4. Nichtwässrige elektrolytische Lösung nach Anspruch 1, wobei ein Konzentrationsverhältnis zwischen $LiPO_2F_2$ und HF (($HF$-Konzentration)/($LiPO_2F_2$-Menge)) in der nichtwässrigen elektrolytischen Lösung 1 : 15.000 bis 1 : 20 beträgt.

5. Energiespeichervorrichtung, umfassend eine positive Elektrode, eine negative Elektrode und eine nichtwässrige elektrolytische Lösung mit einem in einem nichtwässrigen Lösemittel gelösten Elektrolytsalz, wobei die nichtwässrige elektrolytische Lösung die nichtwässrige elektrolytische Lösung nach einem der Ansprüche 1 bis 4 ist.

6. Hochreines Vinylencarbonat, das ein Vinylencarbonat ist, das den Gehalt an Chlorverunreinigungen von Null aufweist, wie durch das Wickbold-Verbrennungslonenchromatographieverfahren nachgewiesen, das an Sauerstoff koordiniert ist, und in einer Stickstoffatmosphäre eine Hazen-Einheit-Farbzahl von 10 oder weniger aufweist, und das, wenn für 7 Tage bei 45 °C in einer sauerstoffhaltigen Atmosphäre gelagert, eine gelbgrüne Farbe in einem Bereich von 10Y bis 10GY, bezüglich eines Tönungskreises des Munsell-Farbsystems, annimmt.

7. Hochreines Vinylencarbonat nach Anspruch 6, das, wenn für 7 Tage bei 45 °C in einer sauerstoffhaltigen Atmosphäre gelagert, einen APHA von 100 aufweist.

8. Hochreines Vinylencarbonat nach Anspruch 6, mit einem Schmelzpunkt bei Atmosphärendruck von 20 °C oder höher und niedriger als 22 °C.

9. Hochreines Vinylencarbonat nach Anspruch 6, wobei die Chlorverunreinigungen, wie durch das Wickbold-Verbrennungs-Ionenchromatographieverfahren nachgewiesen, als in ein Chloratom umgewandelt berechnet werden, auf eine Weise, das eine Probe in einem Lösemittel gelöst und Knallgasflammen-Verbrennungsbehandlung unterzogen wird, wobei das erhaltene Gas in einer wässrigen Natriumcarbonatlösung absorbiert wird und das Chorion in der Absorptionslösung durch Ionenchromatographie gemessen wird.

10. Verfahren zum Produzieren des hochreinen Vinylencarbonats wie in Anspruch 6 definiert, wobei das Vinylencarbonat durch ein Verfahren produziert wird, beinhaltend die folgenden Schritte (A) bis (C):

(A) einen Schritt des Abstreifens von in einem Kristallisationstank kristallisierten Rohvinylencarbonatkristallen durch Verwenden eines Abstreifers und Abscheidens der Vinylencarbonatkristalle in einem Unterteil eines Schmelzreinigungsturms;
(B) einen Schritt des Bringens der abgeschiedenen Vinylencarbonatkristalle und eines Teils der in dem Unterteil des Schmelzreinigungsturms geschmolzenen Vinylencarbonat-Schmelzflüssigkeit in Gegenstromkontakt miteinander; und
(C) einen Schritt des Extrahierens der Vinylencarbonat-Schmelzflüssigkeit aus dem Unterteil des Schmelzreinigungsturms.

11. Verfahren zum Produzieren des hochreinen Vinylencarbonats nach einem der Ansprüche 6 bis 9, umfassend das Bringen von Chlorverunreinigungen enthaltenden Rohvinylencarbonatkristallen und eines Teils einer Schmelzflüssigkeit des Rohvinylencarbonats in Fest-Flüssig-Gegenstromkontakt miteinander.

12. Verfahren zum Produzieren des hochreinen Vinylencarbonats nach Anspruch 11, umfassend die folgenden Schritte (A) bis (C):

(A) einen Schritt des Abstreifens von in einem Kristallisationstank kristallisierten Rohvinylencarbonatkristallen durch Verwenden eines Abstreifers und Abscheidens der Vinylencarbonatkristalle in einem Unterteil eines Schmelzreinigungsturms;

(B) einen Schritt des Bringens der abgeschiedenen Vinylencarbonatkristalle und eines Teils der in dem Unterteil des Schmelzreinigungsturms geschmolzenen Vinylencarbonat-Schmelzflüssigkeit in Gegenstromkontakt miteinander; und

(C) einen Schritt des Extrahierens der Vinylencarbonat-Schmelzflüssigkeit aus dem Unterteil des Schmelzreinigungsturms.

**Revendications**

1. Solution électrolytique non aqueuse présentant un sel d'électrolyte dissous dans un solvant non aqueux, la solution électrolytique non aqueuse comprenant un carbonate de vinylène de haute pureté, dans laquelle le carbonate de vinylène de haute pureté est un carbonate de vinylène présentant la teneur d'impuretés chlorées de zéro telle que détectée par le procédé de combustion Wickbold-chromatographie par échange d'ions, étant coordonné à l'oxygène, et présentant un numéro de couleur en unité Hazen dans une atmosphère d'azote de 10 ou moins, et qui, lorsqu'il est stocké à 45 °C pendant 7 jours dans une atmosphère contenant de l'oxygène, prend une couleur jaune vert dans une plage de 10 Y à 10 GY dans un cercle chromatique du système de couleur Munsell.

2. Solution électrolytique non aqueuse selon la revendication 1, comprenant de 0,1 à 1,5 % en masse de difluorophosphate de lithium dans la solution électrolytique non aqueuse.

3. Solution électrolytique non aqueuse selon la revendication 1, comprenant de 1 à 50 ppm de HF dans la solution électrolytique non aqueuse.

4. Solution électrolytique non aqueuse selon la revendication 1, dans laquelle un rapport en concentration entre $LiPO_2F_2$ et HF ((concentration en HF)/(quantité de $UPO_2F_2$)) dans la solution électrolytique non aqueuse est de 1/15 000 à 1/20.

5. Dispositif de stockage d'énergie comprenant une électrode positive, une électrode négative, et une solution électrolytique non aqueuse présentant un sel d'électrolyte dissous dans un solvant non aqueux, dans lequel la solution électrolytique non aqueuse est la solution électrolytique non aqueuse selon l'une quelconque des revendications 1 à 4.

6. Carbonate de vinylène de haute pureté qui est un carbonate de vinylène présentant la teneur en impuretés chlorées de zéro telle que détectée par le procédé de combustion Wickbold-chromatographie par échange d'ions, étant coordonné à l'oxygène, et présentant un numéro de couleur en unité Hazen de 10 ou moins dans une atmosphère d'azote, et qui, lorsqu'il est stocké à 45 °C pendant 7 jours dans une atmosphère contenant de l'oxygène, prend une couleur jaune vert dans une plage de 10 Y à 10 GY dans un cercle chromatique du système de couleur Munsell.

7. Carbonate de vinylène de haute pureté selon la revendication 6, qui, lorsqu'il est stocké à 45 °C pendant 7 jours dans une atmosphère contenant de l'azote, présente un APHA de 100 ou plus.

8. Carbonate de vinylène de haute pureté selon la revendication 6, présentant un point de fusion à la pression atmosphérique de 20 °C ou supérieur et inférieur à 22 °C.

9. Carbonate de vinylène de haute pureté selon la revendication 6, dans lequel les impuretés chlorées telles que détectées par le procédé de combustion Wickbold-chromatographie par échange d'ions sont calculées comme étant converties en atome de chlore de façon qu'un échantillon soit dissous dans un solvant et soumis à un traitement de combustion à la flamme oxhydrique, le gaz obtenu est absorbé dans une solution aqueuse de carbonate de sodium, et l'ion chlore dans la solution d'absorption est mesuré par chromatographie par échange d'ions.

10. Procédé de production du carbonate de vinylène de haute pureté selon la revendication 6, dans lequel le carbonate de vinylène est produit par un procédé incluant les étapes (A) à (C) suivantes :

(A) une étape de raclage de cristaux de carbonate de vinylène brut cristallisés dans une cuve de cristallisation en utilisant un racloir et de précipitation des cristaux de carbonate de vinylène au fond d'une tour de purification de matière fondue ;
(B) une étape consistant à amener les cristaux de carbonate de vinylène précipités et une partie du liquide fondu de carbonate de vinylène fondu au fond de la tour de purification de matière fondue en contact à contre-courant les uns avec les autre ; et

(C) une étape d'extraction d'une partie du liquide fondu de carbonate de vinylène du fond de la tour de purification de matière fondue.

**11.** Procédé de production du carbonate de vinylène de haute pureté selon l'une quelconque des revendications 6 à 9, comprenant le fait d'amener des cristaux de carbonate de vinylène brut contenant des impuretés chlorées et une partie d'un liquide fondu du carbonate de vinylène brut en contact à contre-courant solide-liquide les uns avec les autres.

**12.** Procédé de production du carbonate de vinylène de haute pureté selon la revendication 11, comprenant les étapes (A) à (C) suivantes :

(A) une étape de raclage de cristaux de carbonate de vinylène brut cristallisés dans une cuve de cristallisation en utilisant un racloir et de précipitation des cristaux de carbonate de vinylène au fond d'une tour de purification de matière fondue ;

(B) une étape consistant à amener les cristaux de carbonate de vinylène précipités et une partie du liquide fondu de carbonate de vinylène fondu au fond de la tour de purification de matière fondue en contact à contre-courant les uns avec les autres ; et

(C) une étape d'extraction d'une partie du liquide fondu de carbonate de vinylène du fond de la tour de purification de matière fondue.

[Fig. 1]

[Fig. 2]

01: Hue circle

1: Red system

2: Yellow-red system

3: Yellow system

4: Yellow-green system

5: Green system

6: Blue-green system

7: Blue system

8: Blue-purple system

9: Purple system

10: Red-purple system

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002008721 A
- JP 2002322171 A
- JP 2008540467 A
- JP 2009029814 A
- JP 2010282760 A
- DE 102005021965 A1
- US 3457279 A
- CN 1789259 A

**Non-patent literature cited in the description**

- Electrolytes for Lithium and Lithium-Ion Batteries. *Modern Aspects of Electrochemistry,* 2014, vol. 58, 172-173
- **SCHNAUTZ, N. G. et al.** Report, PER-11. *Radiation polymerization of vinylene carbonate,* May 1978, 11-6